# EUROPEAN PATENT SPECIFICATION

(11) **EP 2 704 653 B1**
(45) Date of publication and mention of the grant of the patent: **09.09.2015**
(21) Application number: 12716974.6
(22) Date of filing: 16.04.2012
(51) Int. Cl.: A61B 18/02, A61F 7/12, A61B 18/14, A61B 18/00

(54) **ADIABATIC COOLING SYSTEM FOR MEDICAL DEVICES**
ADIABATISCHES KÜHLSYSTEM FÜR MEDIZINISCHE VORRICHTUNGEN
SYSTÈME DE REFROIDISSEMENT ADIABATIQUE POUR DISPOSITIFS MÉDICAUX

(30) Priority: 02.05.2011 US 201113099027
(43) Date of publication of application: 12.03.2014
(73) Proprietor: Medtronic Ablation Frontiers LLC, Minneapolis, MN 55432 (US)
(72) Inventor: ASCONEGUY, Alexander J., Murrieta CA 92563 (US)
(74) Representative: Casey, Lindsay Joseph
(86) International application number: PCT/US2012/033738
(87) International publication number: WO 2012/151038

(56) References cited:
- WO-A1-99/05979
- US-A- 5 688 267
- US-A- 5 899 917
- US-A1- 2008 312 644

## Description

### FIELD OF THE INVENTION

The present invention relates to cooling systems for thermal tissue treatment, and in particular, towards systems for ablating tissue.

### BACKGROUND OF THE INVENTION

A number of cooled catheter systems have been developed for treating tissue in a cardiac setting, either to cool the tissue sufficiently to stun it and allow cold mapping of the heart and/or confirmation of catheter position with respect to localized tissue lesions, or to apply a more severe level of cold to ablate tissue at the site of the catheter ending. In general, these instruments may be configured to achieve either small localized ball shape lesions at the tip of the catheter, or one or more elongated linear lesions extending a length of several centimeters or more along the tip. The latter form of lesion is commonly used to achieve conduction block across a region of the cardiac wall so as to sever an aberrant pathway and prevent conduction across the tissue region in order change the cardiac signal path topology. For example, such a procedure may be implemented to eliminate a faulty pathway responsible for atrial fibrillation or a tachycardia.

An example of a cooled catheter system is provided in document US5688267. A cryogenic device uses the energy transfer derived from thermodynamic changes occurring in the flow of a refrigerant through the device. Various fluids with low operating temperatures (such as cryogens or cryogenic refrigerants) have been used in the medical and surgical field to treat such tissue aberrations. In general, a cryogenic device uses the energy transfer derived from thermodynamic changes occurring in the flow of a cryogen therethrough to create a net transfer of heat flow from the target tissue to the device, typically achieved by cooling a portion of the device to very low temperature through conductive and convective heat transfer between the cryogenic implement and a targeted tissue site. The quality and magnitude of heat transfer is regulated by the device configuration and control of the cryogen flow regime within the device.

Structurally, cooling can be achieved through injection of high pressure refrigerant through an orifice. Upon injection from the orifice, the refrigerant undergoes two primary thermodynamic changes: (i) expanding to low pressure and temperature through positive Joule-Thomson throttling, and (ii) undergoing a phase change from liquid to vapor, thereby absorbing heat of vaporization. The resultant flow of low temperature refrigerant through the device acts to absorb heat from the target tissue and thereby cool the tissue to the desired temperature.

A number of different fluids have been used for the coolant component of cryotreatment catheters, such as a concentrated saline solution or other liquid providing some degree of thermal conductivity and heat capacity. However, typical refrigerants and their respective refrigeration systems may be limited in their thermal conductivity and/or capacity to remove heat, either because of their particular thermal properties or because of insufficient temperature reduction prior to delivery of the refrigerant to a catheter.

To some extent these considerations have been addressed by using a phase change material as the cryogenic fluid, and arranging the catheter such that the phase change, e.g., from a liquid to a gas, occurs in the treatment portion of the catheter tip. Another possible approach is to employ a pressurized gas, and configure the catheter for cooling by expansion of the gas in the tip structure. However, owing to the small size that such a catheter is required to assume for vascular insertion, or the awkwardness of handling a cryogenic treatment probe generally, the design of a safe and effective coolant circulation system which nonetheless dependably provides sufficient cooling capacity at a remote tip and minimizes treatment times while increasing ablative lesion depth and quality remains a difficult goal.

Accordingly, it is desirable to provide efficient medical systems and uses thereof having a cooling capacity that minimizes treatment time while achieving effective depth and quality of treatment.

### SUMMARY OF THE INVENTION

According to an aspect of the present invention, there is provided a medical device as specified in claim 1. According to another aspect of the present invention, there is provided a medical device as specified in any of claims 2 - 11.

The present invention advantageously provides efficient medical systems having a cooling capacity that minimizes treatment time while achieving effective depth and quality of treatment. In particular, a medical device is provided, including an elongate body (such as a flexible catheter for example); and a fluid conduit defining a first segment disposed within the elongate body, a second segment extending from the elongate body, and a third segment adjacent the first segment defining an opening therein. The third segment includes a plurality of windings surrounding the first segment, and the windings include a plurality of openings. The fluid conduit may be malleable, at least partially constructed from a shape memory material, and/or may be selectively transitionable from a first geometric configuration to a second geometric configuration. The device may include an electrode coupled to the fluid conduit; a temperature sensor coupled to the fluid conduit; and/or a coolant supply in fluid communication with the fluid conduit. The elongate body may include an exhaust lumen and the device may include a vacuum source in fluid communication with the exhaust lumen.

A medical treatment system is disclosed, including a catheter body defining a proximal end and a distal end; an ablation element coupled to the catheter body and defining a fluid flow path therethrough, the ablation element including a first segment disposed within the catheter body, a second segment extending out of the distal end of the catheter body, and a third segment coiled around the first segment; and a coolant supply in fluid communication with the fluid flow path. The second segment of the ablation element may have a substantially arcuate shape, and/or the ablation element may be a tube having a substantially contiguous thermally-conductive surface. The system may include an electrode coupled to the ablation element and/or a temperature sensor coupled to the ablation element.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is an illustration of a medical system constructed in accordance with the principles of the present invention;
FIG. 2 is an illustration of a device of the system of FIG. 1; and
FIG. 3 is an illustration of an exemplary use of the system of FIG. 1.

### DETAILED DESCRIPTION OF THE INVENTION

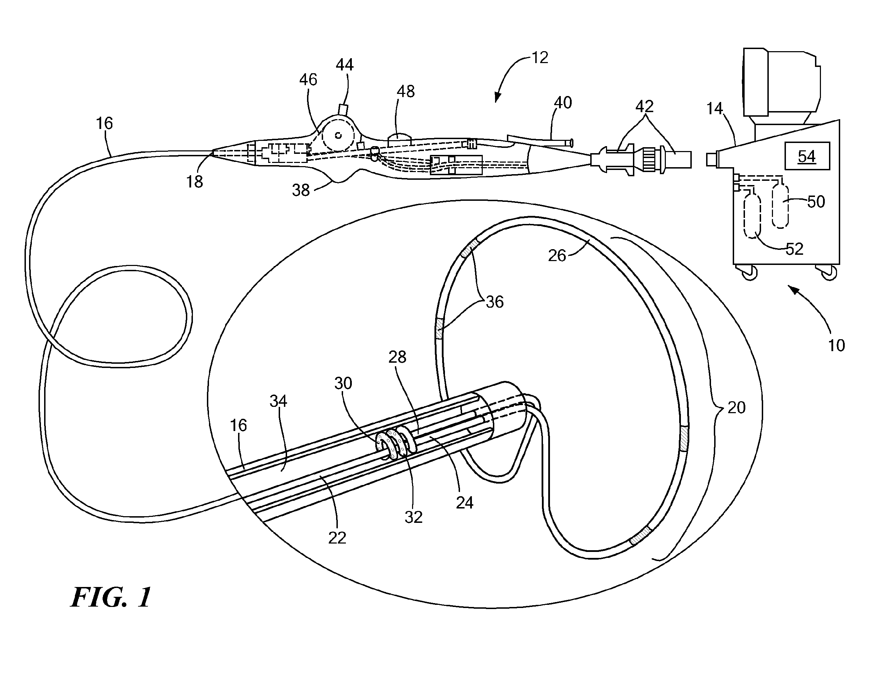

The present invention disclosure advantageously provides efficient medical systems having a cooling capacity that minimizes treatment time while achieving effective depth and quality of treatment. Referring now to the drawing figures in which like reference designations refer to like elements, an embodiment of a medical system constructed in accordance with principles of the present invention is shown in FIG. 1 and generally designated as "10." The system 10 generally includes a medical device 12 that may be coupled to a control unit 14 or operating console. The medical device 12 may generally include one or more diagnostic or treatment regions for energetic, therapeutic and/or investigatory interaction between the medical device 12 and a treatment site. The treatment region(s) may deliver, for example, cryogenic therapy, radiofrequency energy, electroporation treatment or other energetic transfer with a tissue area in proximity to the treatment region(s), including cardiac tissue, tumors, or other undesired growths or structures.

Continuing to refer to FIG. 1, the medical device 12 includes an elongate body 16 passable through a patient's vasculature and/or proximate to a tissue region for diagnosis or treatment, such as a catheter, sheath, or intravascular introducer. The elongate body 16 may define a proximal portion 18 and a distal portion 20, and may further include one or more lumens disposed within the elongate body 16 thereby providing mechanical, electrical, and/or fluid communication between the proximal portion 18 of the elongate body 16 and the distal portion 20 of the elongate body 16, as discussed in more detail below.

The medical device 12 may include a fluid conduit 22 traversing at least a portion of the elongate body. The fluid conduit 22 may include a tube or other structure defining a lumen therein for the passage or delivery of a fluid from the proximal portion of the elongate body 16 and/or the control unit 14 to the distal portion 20 of the medical device 12. The fluid conduit 22 may be at least partially disposed within the elongate body 16 and may also extend from the elongate body 16 or otherwise be exposed at the distal portion 20 of the medical device 12. For example, the fluid conduit defines or includes a first segment 24 disposed within the elongate body 16. The first segment 24 may course along a substantial length of the elongate body 16 and be connectable or otherwise placed into communication with the control unit 14.

The fluid conduit 22 includes or defines a second segment 26 extending from the elongate body 16 at the distal portion 20. The second segment 26 can be positioned directly against a tissue site or anatomical structure for thermal exchange with the tissue. The second segment 26 may be flexible or malleable, and thus provide the ability to transition between one or more selectively controllable geometric configurations. To aid or facilitate the geometric transition, all or a portion of the fluid conduit 22 may be constructed from a shape memory material. For example, as shown in FIG. 1, the second segment 26 of the fluid conduit 22 has a substantially arcuate or circular shape. It is also contemplated that the second segment 26 may also be configured in a substantially linear shape, or virtually any number of other shapes to suit a particular application or tissue region. The second segment 26 may have a substantially contiguous, thermally-conductive region to thermally treat tissue.

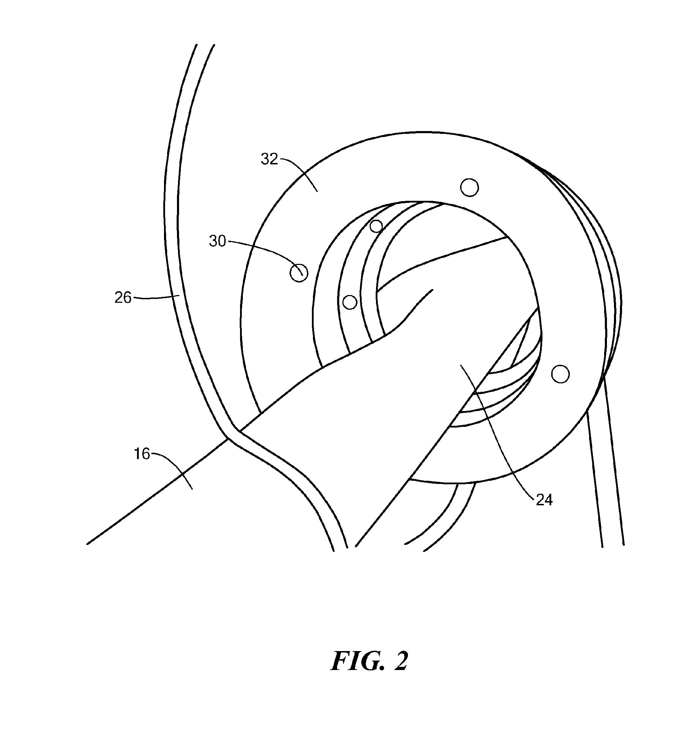

The fluid conduit 22 includes or defines a third segment 28 that is adjacent or in proximity to the first segment of 24 of the fluid conduit, where the third segment 28 includes or defines one or more openings 30 positioned to dispense or otherwise deliver fluid from within the third segment 38 onto an exterior surface or portion of the first segment 24. For example, as shown in FIG. 1, the third segment 28 may be at least partially disposed within the elongate body 16 and oriented substantially parallel to the first segment 24 of the fluid conduit. As shown in FIGS. 1-2, the third segment 28 includes one or more coils or windings 32 that circumscribe or otherwise wrap around a portion of the first segment 24, where the openings 30 are on an interior-facing surface of the windings 32 to direct fluid onto the first segment 24. The windings 32 may be contained within a portion of the elongate body 16, and may be movable and/or slidable with respect to the elongate body 16 other portions of the fluid conduit 22. The windings 32 include one or more of the openings 30 to disperse coolant or a fluid onto the first segment 24 of the fluid conduit 22, where the dispersed coolant or fluid reduces the temperature of the first segment 24 and fluid flowing through it which, in turn, reduces the temperature of the second segment 26, thus allowing it to be used as an ablation element to thermally treat a targeted tissue region.

The fluid conduit 22 may be constructed from one or more materials providing desired conformability and/or geometric transitioning characteristics. For example, a portion of the fluid conduit 22 may be constructed from a substantially rigid polymer or metal (such as a straight segment of the conduit), while a section of the fluid conduit 22 adjacent or in proximity to the distal end (including, for example, the windings or second segment 26) may be constructed from a malleable or shape memory material to readily transition from one configuration to another along with the other components at the distal end.

The medical device 12 may further include an exhaust lumen or conduit 34 providing an evacuation path for fluid dispersed or otherwise present within the medical device 12. The exhaust lumen 34 may be defined by a wall of the elongate body 16 itself, or by another auxiliary tube or conduit disposed within at least a portion of the medical device 12. The exhaust lumen 34 may be in fluid communication with a proximal portion of the elongate body 16 and/or the medical device 12 to allow access and/or coupling of the exhaust lumen 34 to the control unit 14 for removal, storage, and/or recirculation of a fluid exiting the exhaust lumen 34. The exhaust lumen 34 and the fluid conduit 22 may thus provide a fluid flow path through at least a portion of the medical device 12.

The distal portion 20 of the medical device 12 may include one or more electrically conductive portions or electrodes 36 thereon coupled to a radiofrequency generator or power source. In particular, a plurality of electrodes or electrically conductive portions 36 may be disposed on or otherwise situated about the distal portion 20 of the elongate body 16, such as on or about the second segment 26 of the fluid conduit 22. The electrodes 36 may be isolated or separated from the fluid path coursing through the fluid conduit 22.

The medical device 14 may further include one or more temperature sensors (not shown) proximate the distal portion 20 and/or electrodes 36 for monitoring, recording or otherwise conveying measurements or conditions within the medical device 12, the ambient environment at the distal portion 20 of the medical device 12, and/or an interface or junction between the device and a contacted tissue surface. For example, the temperature sensor(s) may include a thermistor coupled to one or more of the electrodes 36; may also be positioned adjacent an electrode 36 at the distal portion 20 of the medical device 12, and/or may be embedded into a surface of the elongate body 16, for example. The sensor(s) may be in communication with the control unit 14 for initiating or triggering one or more alerts or therapeutic delivery modifications during operation of the medical device 12.

Continuing to refer again to FIG. 1, the medical device 12 may include a handle 38 coupled to the proximal portion of the elongate body 16. The handle 38 can include circuitry for identification and/or use in controlling of the medical device 12 or another component of the system 10. Additionally, the handle 38 may be provided with a fitting 40 for receiving a guide wire or another diagnostic/treatment instrument (not shown). The handle 38 may also include connectors 42 that are matable to the control unit 14 to establish communication between the medical device 12 and one or more components or portions of the control unit 14.

The handle 38 may also include one or more actuation or control features that allow a user to control, deflect, steer, or otherwise manipulate a distal portion of the medical device 12 from the proximal portion of the medical device 12. For example, the handle 38 may include one or more components such as a lever or knob 44 for manipulating the elongate body 16 and/or additional components of the medical device 12. For example, a pull wire 46 with a proximal end and a distal end may have its distal end anchored to the elongate body 16 at or near the distal portion 20. The proximal end of the pull wire 46 may be anchored to an element such as a cam in communication with and responsive to the lever 44.

The medical device 12 may include an actuator element 48 that is movably coupled to the proximal portion of the elongate body 16 and/or the handle 38 for the manipulation and movement of a portion of the medical device 12, such as the fluid delivery conduit and/or distal portion, for example. The actuator element 48 may include a thumb-slide, a push-button, a rotating lever, or other mechanical structure for providing a movable coupling to the elongate body 16 and/or the handle. Moreover, the actuator element 48 may be movably coupled to the handle 38 such that the actuator element 48 is movable into individual, distinct positions, and is able to be releasably secured in any one of the distinct positions.

Although illustrated as substantially arcuate or circular, the distal portion 20 of the medical device 12 may be configured into a variety of geometric configurations, as mentioned above. The distal portion 20 and/or related components of the medical device may be flexible or malleable to take on virtually any desired shape. For example, manipulating the shape or configuration of the distal portion 20 may be achieved at least in part through one or more controls at the handle 38 as described herein.

The system 10 may include one or more treatment or diagnostic sources coupled to the medical device 12 for use in an operative procedure, such as tissue ablation, for example. The control unit 14 may include a fluid supply 50 including a coolant, cryogenic refrigerant, or the like, an exhaust or scavenging system (not shown) for recovering or venting expended fluid for re-use or disposal, as well as various control mechanisms. In addition to providing an exhaust function for the fluid or coolant supply, the control unit 14 may also include pumps, valves, controllers or the like to recover and/or re-circulate fluid delivered from the fluid supply 50 to the handle 38, the elongate body 16, and/or the fluid pathway(s) of the medical device 12.

A vacuum pump 52 in the control unit 14 may create a low-pressure environment in one or more conduits within the medical device 12, such as the exhaust lumen 34, so that fluid is drawn into the conduit(s)/lumen(s) of the elongate body 16, away from the distal portion 20 and towards the proximal portion 18 of the elongate body 16.

The console 12 may also include a radiofrequency signal generator or power source 54 in electrical communication with the electrodes 36. The generator 54 may include a plurality of output channels, with each channel coupled to an individual electrode or electrically conductive surface 36 of the medical device 12. The generator 54 may be operable in one or more modes of operation, including for example: (i) bipolar energy delivery between at least two electrodes 30 or electrically-conductive portions of the medical device 12 within a patient's body, (ii) monopolar or unipolar energy delivery to one or more of the electrodes 30 or electrically-conductive portions on the medical device 12 within a patient's body and through a patient return or ground electrode (not shown) spaced apart from the electrodes 36 of the medical device 12, such as on a patient's skin for example, and (iii) a combination of the monopolar and bipolar modes.

The system 10 may further include one or more sensors to monitor the operating parameters throughout the system 10, including for example, pressure, temperature, flow rates, volume, power delivery, impedance, or the like in the control unit 14 and/or the medical device 12, in addition to monitoring, recording or otherwise conveying measurements or conditions within the medical device 12 or the ambient environment at the distal portion of the medical device 12. The sensor(s) may be in communication with the control unit 14 for initiating or triggering one or more alerts or therapeutic delivery modifications during operation of the medical device 12. One or more valves, controllers, or the like may be in communication with the sensor(s) to provide for the controlled dispersion or circulation of fluid through the lumens/fluid paths of the medical device 12. Such valves, controllers, or the like may be located in a portion of the medical device 12 and/or in the control unit 14. The control unit 14 may include one or more controllers, processors, and/or software modules containing instructions or algorithms to provide for the automated operation and performance of the features, sequences, calculations, or procedures described herein.

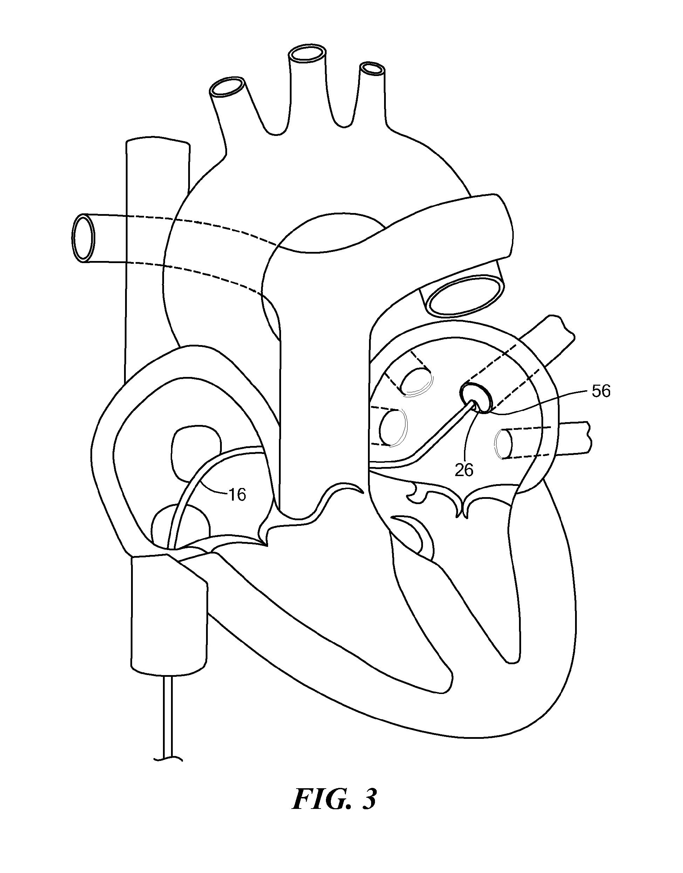

Now referring to FIG. 3, in an exemplary method of use, the medical system 10 may be used to deliver therapeutic treatment, such as ablation treatment for example, to a targeted tissue area 56, which may include a targeted tissue region in the heart, a tumor, or other diagnosed region slated for treatment. The distal portion 20 may be positioned in the proximity of the targeted tissue area 56 and manipulated into the desired geometric configuration, whether circular, arcuate, linear, or the like. Such positioning and manipulation may be aided or facilitated by visualization methods including fluoroscopy or the like as known in the art. Once the medical device 12 is positioned in the desired location, the system 10 may be operated to thermally affect the targeted tissue.

For example, a fluid or coolant may be introduced into the fluid flow path of the medical device 12. In particular, coolant may be transferred from the fluid source 50 in the control unit to the fluid delivery conduit 22. The introduced fluid may be in a substantially liquid phase or state, and be directed distally through the first segment 24 and into the second segment 26 of the fluid conduit 22. The fluid may then flow to the third segment 28 of the fluid conduit 22, where the fluid is ejected or dispersed through one of the ports or openings 30 in the fluid conduit 22. The ejected or dispersed fluid may be directed onto the first segment 24 and may include a phase change from the substantially liquid phase to a substantially gaseous or vapor phase, accompanied by a decrease in temperature. The windings 32 and the dispersed fluid provide thermal exchange between a portion of the fluid conduit 22 downstream of the second segment 26 (e.g., the third segment 28) with portion of the fluid conduit 22 upstream of the second segment 26 (i.e., the first segment 24), resulting in a subcooling effect for fluid passing through the first segment 24 and to the second segment 26, which may then be used to thermally treat or ablate tissue.

The fluid flow through the fluid conduit 22 of the medical device 12 may be intermittently provided in pulsed flow, or may be continuously circulated throughout the segments of the conduit 22. Further, the control unit 14 may modulate fluid flow by varying pressure, flow rates, or the like in direct response to the measured temperatures in the distal portion 20 as relayed by the temperature sensors. For example, upon reaching a preselected temperature threshold or range at the tissue interface, fluid flow through the fluid flow path may be increased or decreased accordingly to substantially maintain a target temperature at the distal portion 20 of the device 12.

In addition and/or alternatively to thermal treatment with the second segment 26, the electrodes 36 may deliver radiofrequency energy treatment to the targeted tissue to achieve the desired therapeutic effect, such as the controlled ablation of problematic tissue to an effective depth within the targeted tissue region. Powering of the electrodes 36 may include delivery of a radio frequency signal or current form the radiofrequency generator 54 resulting in a current flow, and thus heating, between one or more of the electrodes 36 either between each other (e.g., bipolar RF delivery) or to a ground/patient electrode (not shown) in unipolar or monopolar operation. The electrodes 36 may be powered ablate or otherwise treat tissue until a preselected temperature or power delivery threshold has been reached. The predefined temperature or power delivery threshold may be selected to ensure that the affected tissue is not charred or otherwise heated to an undesirable degree.

It will be appreciated by persons skilled in the art that the present invention is not limited to what has been particularly shown and described herein above. In addition, unless mention was made above to the contrary, it should be noted that all of the accompanying drawings are not to scale. Of note, the device components have been represented where appropriate by conventional symbols in the drawings, showing only those specific details that are pertinent to understanding the embodiments of the present invention so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the system and devices disclosed herein are not necessarily exclusive of each other and may be included in a variety of different combinations or configurations without departing from the scope of the invention. Indeed, a variety of modifications and variations are possible in light of the above teachings without departing from the scope of the invention, which is limited only by the following claims.

## Claims

1. A medical device (12), comprising:
an elongate body (16); and
a fluid conduit (22) defining a first segment (24) disposed within the elongate body (16), a second segment (26) extending from the elongate body (16), and a third segment (28) adjacent to the first segment (24) within the elongate body (16),
**characterised by** the third segment (28) including a plurality of windings (32) surrounding the first segment (24), the plurality of windings (32) defining at least one opening (30) to dispense fluid onto the surface of the first segment (24).

2. The medical device (12) of claim 1, wherein the fluid conduit (22) is at least one of substantially malleable or constructed from a shape memory material.

3. The medical device (12) of claim 1, wherein the fluid conduit (22) is selectively transitionable from a first geometric configuration to a second geometric configuration.

4. The medical device (12) of claim 1, wherein the elongate body (16) is a flexible catheter.

5. The medical device (12) of claim 1, further comprising an electrode coupled to the fluid conduit (22).

6. The medical device (12) of claim 1, further comprising a temperature sensor coupled to the fluid conduit.

7. The medical device (12) of claim 1, further comprising a coolant supply in fluid communication with the fluid conduit (22).

8. The medical device (12) of claim 1, wherein the elongate body (16) includes an exhaust lumen (34).

9. The medical device (12) of claim 8, further comprising a vacuum source in fluid communication with the exhaust lumen (34).

10. The medical device (12) of claim 1, wherein the second segment defines a substantially arcuate shape.

11. The medical device (12) of claim 1, wherein the fluid conduit (22) defines a substantially contiguous thermally-conductive surface (36).

## Patentansprüche

1. Medizinische Vorrichtung (12), die Folgendes umfasst:
einen langgestreckten Körper (16); und
eine Fluidleitung (22), die ein in dem langgestreckten Körper (16) angeordnetes erstes Segment (24), ein sich von dem langgestreckten Körper (16) erstreckendes zweites Segment (26) und ein dem ersten Segment (24) benachbartes drittes Segment (28) in dem langgestreckten Körper (16) definiert, **dadurch gekennzeichnet, dass** das dritte Segment (28) mehrere das erste Segment (24) umgebende Windungen (32) umfasst,
wobei die mehreren Windungen (32) mindestens eine Öffnung (30) definieren, um Fluid auf die Oberfläche des ersten Segments (24) auszugeben.

2. Medizinische Vorrichtung (12) nach Anspruch 1, wobei die Fluidleitung (22) im Wesentlichen umformbar und/oder aus einem Formgedächtnismaterial aufgebaut ist.

3. Medizinische Vorrichtung (12) nach Anspruch 1, wobei die Fluidleitung (22) selektiv von einer ersten geometrischen Konfiguration in eine zweite geometrische Konfiguration übergehen kann.

4. Medizinische Vorrichtung (12) nach Anspruch 1, wobei es sich bei dem langgestreckten Körper (16) um einen biegsamen Katheter handelt.

5. Medizinische Vorrichtung (12) nach Anspruch 1, weiter umfassend eine an die Fluidleitung (22) gekoppelte Elektrode.

6. Medizinische Vorrichtung (12) nach Anspruch 1, weiter umfassend einen an die Fluidleitung gekoppelten Temperatursensor.

7. Medizinische Vorrichtung (12) nach Anspruch 1, weiter umfassend eine Kühlmittelversorgung in Fluidverbindung mit der Fluidleitung (22).

8. Medizinische Vorrichtung (12) nach Anspruch 1, wobei der langgestreckte Körper (16) ein Auslasslumen (34) umfasst.

9. Medizinische Vorrichtung (12) nach Anspruch 8, weiter umfassend eine Vakuumquelle in Fluidverbindung mit dem Auslasslumen (34).

10. Medizinische Vorrichtung (12) nach Anspruch 1, wobei das zweite Segment eine im Wesentlichen bogenförmige Form definiert.

11. Medizinische Vorrichtung (12) nach Anspruch 1, wobei die Fluidleitung (22) eine im Wesentlichen zusammenhängende wärmeleitfähige Oberfläche (36) definiert.

## Revendications

1. Dispositif médical (12), comportant :
un corps allongé (16) ; et
un conduit pour fluide (22) définissant un premier segment (24) disposé à l'intérieur du corps allongé (16), un deuxième segment (26) s'étendant en provenance du corps allongé (16), et un troisième segment (28) adjacent par rapport au premier segment (24) à l'intérieur du corps allongé (16),
**caractérisé par** le troisième segment (28) comprenant une pluralité d'enroulements (32) entourant le premier segment (24), la pluralité d'enroulements (32) définissant au moins une ouverture (30) à des fins de distribution du fluide sur la surface du premier segment (24).

2. Dispositif médical (12) selon la revendication 1, dans lequel le conduit pour fluide (22) est au moins l'un parmi sensiblement malléable ou construit à partir d'un matériau à mémoire de forme.

3. Dispositif médical (12) selon la revendication 1, dans lequel le conduit pour fluide (22) est en mesure de passer de manière sélective d'une première configuration géométrique à une deuxième configuration géométrique.

4. Dispositif médical (12) selon la revendication 1, dans lequel le corps allongé (16) est un cathéter souple.

5. Dispositif médical (12) selon la revendication 1, comportant par ailleurs une électrode couplée au conduit pour fluide (22).

6. Dispositif médical (12) selon la revendication 1, comportant par ailleurs un capteur de température couplé au conduit pour fluide.

7. Dispositif médical (12) selon la revendication 1, comportant par ailleurs une alimentation en fluide de refroidissement en communication fluidique avec le conduit pour fluide (22).

8. Dispositif médical (12) selon la revendication 1, dans lequel le corps allongé (16) comprend une lumière d'évacuation (34).

9. Dispositif médical (12) selon la revendication 8, comportant par ailleurs une source de vide en communication fluidique avec la lumière d'évacuation (34).

10. Dispositif médical (12) selon la revendication 1, dans lequel le deuxième segment définit une forme sensiblement arquée.

11. Dispositif médical (12) selon la revendication 1, dans lequel le conduit pour fluide (22) définit une surface thermoconductrice sensiblement contiguë (36).
